# EUROPEAN PATENT APPLICATION

(11) **EP 0 734 726 A2**
(43) Date of publication of application: **02.10.1996**
(21) Application number: 96104821.2
(22) Date of filing: 27.03.1996
(51) Int. Cl.: A61K 33/28

(54) **Composition containing mercury ammonium chloride, resorcinol and sulphur**

(30) Priority: 28.03.1995 IT MI950625
(71) Applicant: Joudioux, Bruno, 80100 Napoli (IT); Joudioux, Claudio, 80100 Napoli (IT)
(72) Inventor: Joudioux, Bruno, 80100 Napoli (IT); Joudioux, Claudio, 80100 Napoli (IT)
(74) Representative: Beneduce, Gianna

(57) **Abstract**

A pharmaceutical composition possessing activity in the cure of dermatological affection, in particular, in the cure of psoriasis, parapsoriasis, eczema and scabies is described.

## Description

The present invention relates to a pharmaceutical composition active in the cure of dermatological affections.

More in particular said composition is suitable for topical administration and it is active in many pathologies of dermatological kind, such as, in particular, psoriasis, parapsoriasis, eczema and scabies.

Among the above said pathologies, psoriasis is certainly one of the cutaneous illnesses mainly diffused and also because of its not known etiology and of its natural persistence, at least in the most severe forms, the normally employed medicaments have had a palliative effect with frequent relapses.

It affects mainly, without any sex distinction, medium aged persons, while the younger and the older result to be quite spared.

The psoriasis distinguishes itself with cutaneous manifestations of scale-erythematous type, more specifically with white coloured spots which show a redness at the circumference; said spots are prevalently located on the anterior face of the lower limbs and on the posterior face of the upper limbs, in particular, on knees and elbows.

Since the etiology of the psoriasis is not known, the therapy which is followed for the cure of said affection does not face the cause, but it limits itself to the release of the symptoms: therefore, persisting the cause which provokes it, the illness almost always relapses.

Traditionally the therapy by local administration is considered the best for the cure of psoriasis: this is conducted by first using suitable keratolytic compounds by which the scales in the affected area are removed, then provoking a reaction of the treated skin by means of reducing agents. For this purpose, as keratolytic agents, suitable concentrations of salicylic acid and, as reducing agents ichthyol, tar, pyrogallic acid, crysarobine and cignoline, these last two substances being particularly suitable, may be used.

The above therapy, depending on the substances which are used, may require to be carried out in hospital both because of the nature of said substances which requires a continuos medical control of the patient and because said substances may be extremely staining.

It is also known the use of derivatives of mercury, specifically the mercury ammonium chloride or white mercuric precipitate, HgCl.NH₂, as active principle in preparations for topical use; these preparations have been used in the psoriasis therapy in particular in the East Europe Countries.

More recently in the cure of psoriasis, antiinflammatory and immunosuppressive cortisone compounds have been used, such as cyclosporine A. Also heliotherapy and UVB therapy have been used.

However it is to be pointed out that none of the therapies followed up to today has conducted to a real recovery of the illness.

It has now been prepared a pharmaceutical composition suitable for topical administration, constituted by suitably mixed mercury ammonium chloride, resorcinol and sulphur, together with suitable conventional eccipients, which has demonstrated to be very active in the cure of psoriasis, parapsoriasis, eczema and scabies.

In particular in the case of psoriasis, the pharmaceutical composition of the present invention, in respect to the active principles up to today used, included mercury ammonium chloride as such, has evidenced a stronger and more specific activity which has allowed to cure said pathologies without evidencing after the end of the treatment, any relapse.

In the pharmaceutical composition of the present invention mercury ammonium chloride, resorcinol and sulphur components, are suitably mixed with conventional eccipients suitable for the preparation of pharmaceutical preparations for topical use, such as creams, emulsions, gel, ointments. Depending also from the type of pharmaceutical preparations used, the composition of the present invention may preferably contain 1-7% by weight of mercury ammonium chloride, 0.7-3% by weight of resorcinol and 0.1-2% by weight of sulphur.

In a preferred formulation of said composition the components are contained in the following ratio:

| | |
|---|---|
| mercury ammonium chloride | 5 g |
| resorcinol | 2 g |
| sulphur | 1 g |
| pure vasalline | 100 g |
| talc | q.b. |

The ointment so obtained was administered to a group of 15 voluntaries affected by psoriasis, aged 20 up to 45 years, by daily occlusive treatment.

After 15 days of treatment a marked improvement of the conditions was registered in all the patients treated while in the next 30 days a complete remission of the pathology was obtained. During the treatments, the patients did not feel any type of burning sensation or inflammation in the skin area treated.

## Claims

1. Topical pharmaceutical composition active in dermatological affection, in psoriasis, parapsoriasis, eczema and scabies, characterized by the fact that it is formed by a suitable mixture of mercury ammonium chloride, resorcinol and sulphur suitably mixed with proper pharmaceutically acceptable excipients.

2. Topical pharmaceutical composition active against psoriasis, characterized by the fact that it is formed by a suitable mixture of mercury ammonium chloride, resorcinol and sulphur suitably mixed with proper pharmaceutically acceptable excipients.

3. Pharmaceutical composition according to claim 2, characterized by the fact that it contains 1-7% by weight of mercury ammonium chloride, 0.7-3% by weight of resorcinol and 0.1-2% by weight of sulphur.

4. Pharmaceutical composition according to claim 3, characterized by the fact that it contains 5 g of mercury ammonium chloride, 2 g resorcinol, 1 g sulphur, 100 g pure vaseline and talc q.s.
